Europäisches Patentamt

European Patent Office

Office européen des brevets

(19)

(11) Numéro de publication : **0 069 037**
**B1**

(12) **FASCICULE DE BREVET EUROPÉEN**

(45) Date de publication du fascicule du brevet :
07.05.86

(51) Int. Cl.⁴ : **A 61 L 2/26, C 12 Q 1/22**

(21) Numéro de dépôt : **82430015.6**

(22) Date de dépôt : **11.06.82**

(54) **Indicateur de stérilisation.**

(30) Priorité : **26.06.81 FR 8112788**

(43) Date de publication de la demande :
**05.01.83 Bulletin 83/01**

(45) Mention de la délivrance du brevet :
**07.05.86 Bulletin 86/19**

(84) Etats contractants désignés :
**AT BE CH DE GB IT LI LU NL SE**

(56) Documents cités :
**FR-A- 1 461 252**
**FR-A- 2 307 544**
**GB-A- 1 215 891**
**US-A- 3 107 204**
**US-A- 3 862 824**
**US-A- 4 091 921**

(73) Titulaire : **Charvin, Guy**
**Parc Saint Honoré Chemin de la Peyrigoue**
**F-06600 Antibes (FR)**

(72) Inventeur : **Charvin, Guy**
**Parc Saint Honoré Chemin de la Peyrigoue**
**F-06600 Antibes (FR)**

(74) Mandataire : **Azais, Henri et al**
**c/o CABINET BEAU DE LOMENIE 14, rue Raphael**
**F-13008 Marseille (FR)**

Jouve, 18, rue St-Denis, 75001 Paris, France

## Description

La présente invention a pour objet des indicateurs de stérilisation du type comportant des taches colorées dont le changement de couleur indiquent une bonne stérilisation et qui permettent de contrôler l'efficacité des opérations de stérilisation à la vapeur d'eau sous pression.

On connaît des indicateurs de stérilisation composés d'un petit tube transparent et clos dans lequel est placé un indicateur coloré liquide. Ces indicateurs sont placés dans des autoclaves de stérilisation et lorsque la température et la durée nécessaires pour une bonne stérilisation ont été atteintes, le liquide change de couleur. Ces indicateurs à tube ne peuvent pas tenir compte de la présence ou de l'absence de vapeur d'eau saturée qui est un facteur important de la stérilisation, notamment quand il s'agit de stériliser des paquets de linge tels que des champs opératoires, des vêtements de chirurgie, des draps etc....

On connaît d'autres indicateurs de stérilisation qui sont composés d'un support ayant la forme d'une plaquette ou d'une bande, en papier, en carton ou en un autre matériau absorbant sur laquelle sont disposées des taches d'un réactif physico-chimique, qui changent de couleur dans les conditions de la stérilisation. Ces indicateurs peuvent comporter plusieurs taches et ils peuvent donner des indications sur la température, la pression et la durée de la stérilisation ainsi que sur la présence de vapeur d'eau.

Le brevet US-1 894 015 (W. Bernstein) décrit de tels indicateurs de stérilisation comportant des zones colorées formées d'un mélange pâteux de soufre, d'un composé de plomb et de différents catalyseurs qui réagissent en présence de vapeur d'eau à une température déterminée pour former des composés de couleur déterminée.

Le brevet US-2 118 144 (P. Berman et al) décrit des indicateurs de stérilisation comportant des taches d'encre dans laquelle est incorporé un mélange de soufre et d'un composé de plomb tel que l'oxyde de plomb ou litharge.

D'autres indicateurs de stérilisation connus comportent des taches d'un réactif contenant du chlorure de chrome.

Les réactifs entrant dans la composition des taches colorées des indicateurs connus à ce jour sont agressifs, notamment à l'égard des tissus et l'expérience a montré que si un indicateur de stérilisation était placé à l'intérieur d'un paquet de linge, dans un autoclave de stérilisation, des trous apparaissaient dans les parties de linge qui avaient été placées au contact des taches colorées.

Les indicateurs contenant du chlorure de chrome se décomposent en libérant du chlore qui donne naissance, en présence de vapeur d'eau, à de l'acide chlorhydrique très agressif.

De plus, il peut être dangereux que les réactifs viennent au contact des objets à stériliser par exemple des instruments chirurgicaux.

Une solution pour éviter ces accidents consiste à placer chaque indicateur coloré dans un étui perforé ou à claire-voie, qui évite le contact direct entre le linge et les taches colorées. Cette solution est peu pratique. Elle conduit à des accidents du fait que les opérateurs peuvent oublier de placer l'indicateur dans l'étui. De plus, les indicateurs colorés sont des objets de faible prix, à jeter après usage. Généralement, on insère un indicateur dans chaque paquet de linge avant stérilisation et cet indicateur reste dans le paquet jusqu'à ce que celui-ci soit ouvert par l'utilisateur, par exemple un service de chirurgie d'un hôpital, qui peut ainsi contrôler que la stérilisation a eu lieu. Dans ce cas, les services utilisateurs omettent fréquemment de retourner les étuis au service de stérilisation, ce qui accroît très sensiblement le coût d'utilisation de ces indicateurs colorés.

La publication FR-A-2 307 544 (Bio-Médical Sciences Inc.) décrit des indicateurs de stérilité comportant une bande-support revêtue d'une couche d'adhésif et portant une pastille d'un composé organique, une mèche et une bande de couverture transparente, qui est perméable à la vapeur d'eau et qui est liée par adhérence à la face supérieure de la bande-support.

Le brevet US-3 862 824 (A. Chapman) décrit des indicateurs de stérilisation par la vapeur comportant un support sur lequel est déposé un mélange d'acide carboxylique et de l'un de ses sels et un indicateur de PH et ce support est scellé dans une enveloppe en matière plastique transparente qui est perméable à la vapeur d'eau.

Le brevet GB-1 215 891 (R. Maxwell) décrit des indicateurs de stérilisation par la vapeur qui comportent un support opaque sur lequel sont déposés un indicateur sensible à la vapeur et un indicateur sensible à la température, lequel support est recouvert d'une feuille transparente et perméable à la vapeur.

L'objectif de la présente invention est de procurer des indicateurs de stérilisation du type comportant des taches de couleur variable placées sur une plaquette-support, qui comportent un écran peu onéreux incorporé à chaque indicateur et jetable avec celui-ci, lequel écran évite le contact direct entre les taches colorées et les objets à stériliser, notamment avec le linge lorsqu'ils sont placés dans un paquet de linge, tout en permettant un contact suffisant entre l'atmosphère du stérilisateur et les taches colorées pour que le gaz stérilisant puisse parvenir au contact des taches colorées et que les durées de virage des couleurs restent sensiblement les mêmes que celles des indicateurs sans écran.

Les indicateurs de stérilisation selon l'invention sont du type connu comportant une plaquette-support, dont au moins l'une des faces porte une ou plusieurs taches d'un réactif coloré qui change de couleur en fonction de la température, de la pression, de la concentration en vapeur d'eau et/ou de la durée de séjour dans un stérilisateur.

Ils comportent, de façon connue, un écran constitué par un film transparent qui est imperméable aux liquides engendrés par l'opération de stérilisation et qui recouvre entièrement les taches de réactif.

L'objectif de l'invention est atteint au moyen d'un indicateur de stérilisation dans lequel l'écran transparent est constitué par un film complexe composé d'une feuille de polypropylène ou de polyuréthane à l'intérieur et d'une feuille de polyester ou de polyamide à l'extérieur, lequel film est fixé par thermofusion, de façon discontinue et non étanche, à ladite plaquette-support, de sorte que la vapeur d'eau peut passer entre ledit écran et ladite plaquette-support.

Selon un mode de réalisation, la plaquette-support est une bande rectangulaire et ledit écran a également la forme d'une bande rectangulaire qui est superposée à la face de la plaquette portant les taches de réactif et qui est fixée à ladite face par deux de ses bords opposés.

Avantageusement, la bande transparente a les mêmes dimensions que la plaquette-support et elle est fixée aux petits côtés de la plaquette-support par ses deux petits côtés.

Selon un autre mode de réalisation, la plaquette-support est constituée d'un carton ondulé permettant le passage latéral de la vapeur d'eau à travers les canaux formés par les ondulations de la feuille de carton intérieure.

L'invention a pour résultat de nouveaux indicateurs de stérilisation comportant un écran transparent incorporé. L'écran transparent permet de voir les changements de couleur par transparence. Il évite le contact direct d'attaque des tissus. Il évite également que des traces du réactif coloré, qui peuvent être toxiques, ne risquent de souiller un objet en cours de stérilisation par exemple un instrument chirurgical.

Du fait que l'écran transparent est fixé de façon discontinue et non étanche, la vapeur d'eau peut atteindre efficacement les taches de réactif, de telle sorte que les changements de couleur du réactif se produisent pratiquement dans les mêmes conditions que pour un indicateur sans écran.

Par contre, le liquide de condensation qui peut se former sous l'écran par la réaction de la vapeur d'eau saturée avec le réactif coloré, ne peut traverser l'écran qui est imperméable aux liquides engendrés par l'opération de stérilisation, de sorte qu'il n'y a plus de risque de contact des objets placés dans l'étuve avec un liquide agressif.

Le fait que l'écran transparent est un film complexe permet que la face interne en polyuréthane ou en polypropylène fond et peut être thermosoudée partiellement à la plaquette de carton et que la face externe en polyester ou en polyamide ne fond pas à la température des électrodes de soudure, ce qui évite que l'écran ne reste collé aux électrodes et qu'il ne risque de se déchirer.

La description suivante se réfère aux dessins annexés qui représentent, sans aucun caractère limitatif, des exemples de réalisation d'indicateurs de stérilisation selon l'invention.

La figure 1 est une vue en perspective d'un premier mode de réalisation d'un indicateur de stérilisation selon l'invention.

La figure 2 est une vue en perspective d'un deuxième mode de réalisation d'un indicateur de stérilisation selon l'invention.

Les figures 3 à 7 sont des coupes longitudinales de variantes de réalisation.

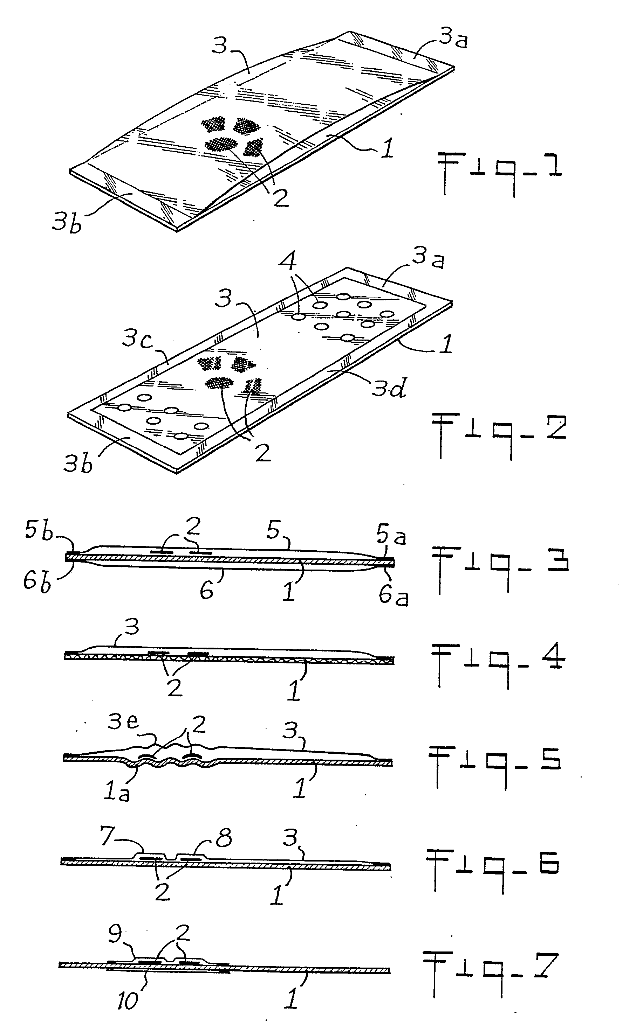

La figure 1 représente un indicateur de stérilisation qui est composé d'une plaquette-support 1, ayant la forme d'une bande rectangulaire, en papier buvard ou en carton mince, ou en tout autre matériau équivalent. Sur cette plaquette sont disposées des taches de réactif 2 qui changent de couleur lorsque l'indicateur est placé dans un stérilisateur, par exemple dans un autoclave de stérilisation par la vapeur d'eau saturée et qu'il a été soumis aux conditions qui assurent une bonne stérilisation. Les changements de couleur des taches de réactif 2 en fin d'opération permettent de contrôler l'efficacité de la stérilisation.

L'indicateur comporte, en outre, un écran transparent 3 qui est constitué par un film en matière plastique qui recouvre entièrement les taches de réactif 2 et qui est fixé de façon non étanche à la plaquette 1.

Dans l'exemple de la figure 1, l'écran 3 a la forme d'une bande transparente identique à la plaquette et cette bande est fixée à la plaquette, par deux zones 3a, 3b situées le long des petits côtés de la plaquette 1 et de la bande 3. Grâce à ce mode de fixation discontinu, la vapeur d'eau ou le gaz peut pénétrer entre l'écran 3 et la plaquette 1 pour atteindre normalement les taches de réactif 2.

Si l'indicateur selon la figure 1 est introduit au centre d'un paquet de linge en cours de stérilisation, qui est l'endroit le plus important à contrôler, le linge ne peut se trouver en contact avec les taches de réactif mais la vapeur d'eau peut atteindre celles-ci.

En variante, les bandes transparentes 3 pourraient être plus courtes que les plaquettes 1 et être fixées par leurs petits côtés de part et d'autre de la plage où se trouvent les taches 2.

Selon une autre variante, les bandes 3 peuvent être fixées à la plaquette 1 uniquement par leurs grands côtés ou par trois de leurs côtés.

La figure 2 représente une variante d'un indicateur de stérilisation comportant une plaquette 1, des taches de réactif 2 et un écran transparent 3, qui est fixé à la plaquette par quatre zones 3a, 3b, 3c, 3d situées le long des quatre côtés et qui comporte des perforations 4, qui sont situées en dehors des zones qui recouvrent les taches 2. La vapeur d'eau ou le gaz pénètrent par les perforations 4 pour atteindre les taches de réactif 2.

La figure 3 représente une autre variante de réalisation dans laquelle les deux faces de la plaquette 1 portant les taches de réactif 2 sont recouvertes d'un écran transparent respectivement un écran 5 fixé en 5a et 5b et un écran 6, fixé en 6a et 6b. Cette figure représente un mode de réalisation préférentiel dans le cas où la plaquette-support 1 est en carton ou papier très absorbant ou poreux et où le réactif 2 risque de migrer à travers le support en présence de vapeur d'eau saturée. Dans ce cas, il est préférable de recouvrir les deux faces du support 1 par un écran afin d'éviter des risques d'accident. L'écran 5 qui recouvre les taches 2 est transparent et il est fixé, de façon discontinue à la face supérieure de la plaquette 1. Par contre, l'écran 6 peut être opaque et il peut être fixé, soit de façon continue et étanche à la plaquette, soit de façon discontinue.

La figure 4 représente une autre variante de réalisation dans laquelle la plaquette 1 est composée d'un carton ondulé très mince portant les taches de réactif 2 sur sa face supérieure. Cette face est recouverte par un écran transparent 3. Dans ce cas, l'écran 3 peut être fixé à la plaquette 1 sur une plus grande partie de son pourtour car la vapeur d'eau ou le gaz pénètrent latéralement dans les ondulations du carton et atteignent les taches de réactif 2 en traversant le carton microporeux.

Les expériences qui ont été réalisées ont montré que les interstices entre l'écran 3 et la plaquette-support, dus à la fixation discontinue de l'écran 3 ou aux perforations 4, sont suffisants pour permettre le passage de la vapeur d'eau du fait notamment des différents coefficients de dilatation des matériaux constituant généralement les indicateurs (plastique pour le film, carton pour la plaquette-support) qui entraînent des déformations de l'indicateur laissant un passage sous l'écran 3 pour la circulation de la vapeur. D'autre part, cette pénétration de vapeur est accélérée par la mise sous vide préalable des autoclaves modernes, éliminant l'air avant la phase d'injection de la vapeur dans le stérilisateur.

Pour faciliter le passage de la vapeur d'eau sous l'écran transparent 3, on peut gaufrer une zone 1a de la plaquette portant les taches de réactif 2 et/ou une zone 3e de l'écran qui recouvre les taches 2, comme le représente la figure 5. Ce gaufrage est obtenu par exemple en conformant les plaquettes et les écrans entre deux mâchoires d'une presse qui comportent des surfaces non unies qui impriment des creux et des reliefs. On peut également remplacer le gaufrage par des stries transversales.

La figure 6 représente une autre variante, dans laquelle l'écran transparent 3 est constitué par une feuille en matière plastique qui est thermoformée dans la zone qui recouvre les taches de réactif 2 pour former un ou plusieurs alvéoles 7, 8 qui épousent le contour des taches de réactif 2. Les alvéoles 7, 8 sont prolongés latéralement par des canaux qui débouchent le long des bords longitudinaux de l'écran 3 et qui facilitent le passage de la vapeur d'eau.

L'écran 3 est en une matière plastique transparente qui résiste à une température de 140 °C, qui est la température la plus élevée dans les stérilisations courantes à la vapeur d'eau.

Selon l'invention, l'écran 3 est composé d'un film complexe comportant une feuille en polypropylène ou polyuréthane à l'intérieur, c'est-à-dire du côté de la plaquette et une feuille extérieure en polyester ou en polyamide. Ce mode de réalisation permet de fixer l'écran transparent à la plaquette-support par thermoscellage entre des électrodes chauffantes portées à une température de l'ordre de 180° à 200 °C. Le polypropylène ou le polyuréthane, fondent et pénètrent dans les matériaux de la plaquette. A la température de fusion du film intérieur, le film extérieur en polyester ou en polyamide ne fond pas. De ce fait, il sert d'agent de maintien de l'écran lorsque la face interne est à l'état de fusion et, de plus, il évite que l'écran ne reste collé aux électrodes de soudure.

L'épaisseur de l'écran transparent peut être très faible, de l'ordre de 5 $\mu$ à 100 $\mu$, de sorte que ces écrans sont peu onéreux en matière et en frais de fabrication et ils n'accroissent pas sensiblement le coût des indicateurs qui sont des objets jetables après usage.

La figure 7 représente une autre variante dans laquelle l'écran transparent comporte un film microporeux 9 qui recouvre les taches de réactif. Le film microporeux 9 peut être thermoscellé à la plaquette-support sur toute sa périphérie. Il peut également être fixé à la plaquette-support sur toute sa surface par thermosoudage, par collage ou par enduction de la face supérieure de la plaquette au moyen d'une résine qui est appliquée à l'état liquide ou visqueux avant polymérisation ou réticulation. La figure 7 représente un mode de réalisation dans lequel un deuxième film 10, étanche ou microporeux, est fixé au verso de la zone de la plaquette portant les taches de réactif.

Des essais ont montré que des films microporeux ayant une perméabilité suffisante à la vapeur d'eau permettent d'obtenir une concentration suffisante de vapeur d'eau sur les taches de réactif de telle sorte que les changements de couleur du réactif se produisent pratiquement dans les mêmes conditions que pour des indicateurs sans écran, notamment dans le cas le plus fréquent où les indicateurs comportent un réactif contenant du chlorure de chrome.

On a pu mesurer la perméabilité minima à la vapeur d'eau qui permet d'obtenir ce résultat et on a trouvé qu'il fallait utiliser des films microporeux ayant une perméabilité à la vapeur d'eau d'au moins 100 g/m²/24H mesurée à une température de 38 °C, dans une atmosphère contenant une humidité relative de 90 %. La méthode de mesure de perméabilité à la vapeur d'eau qui a été utilisée est celle qui est définie dans l'ouvrage intitulé : Les Cahiers Documentaires édités par l'Institut Français de l'Emballage et du Conditionnement dans un article intitulé : « Perméabilité des films et matériaux souples », par A. Buquet et Ph. Manchon.

Le mode de réalisation utilisant un écran constitué par un film microporeux est particulièrement avantageux car le film est suffisamment perméable à la vapeur d'eau, pour que le changement de couleur du réactif intervienne dans des conditions pratiquement inchangées, et, en même temps, le film est imperméable aux liquides et il fixe d'une manière indélébile les taches de réactif en les empêchant de se dissoudre et de s'étendre à la surface de la plaquette-support et, éventuellement, d'être entraînées au contact des linges et instruments placés dans le stérilisateur en cas d'une éventuelle condensation de vapeur d'eau sur la plaquette.

Avantageusement, on utilise comme écran transparent un film d'un type utilisé comme pansement adhésif, notamment un pansement adhésif composé d'un film microporeux en polyuréthane ayant par exemple une épaisseur de l'ordre de 28 μ et d'une couche d'adhésif perméable à la vapeur d'eau, ayant une épaisseur de l'ordre de 38 μ, et composée d'un mélange d'éthers vinyliques et/ou d'esters acryliques.

Un tel film adhésif a une perméabilité à la vapeur d'eau de 800 g/m²/24H à 37 °C sous une humidité relative de 85 %, donc une perméabilité nettement supérieure à la limite minima.

La présente invention constitue une application nouvelle des films adhésifs dans un domaine différent de celui pour lequel ils sont fabriqués. De façon générale, on peut utiliser comme écran microporeux tous les pansements adhésifs transparents qui sont décrits dans la demande de brevet FR 2 012 584 (T.J. Smith et Nephew Lted) et qui présentent une thermorésistance suffisante entre 115 °C et 140 °C.

Les indicateurs de stérilisation portant des taches de réactif coloré contenant du chlorure de chrome sont très utilisés comme indicateurs dans les autoclaves où la stérilisation a lieu en présence de vapeur d'eau sous pression.

La décomposition du chlorure de chrome peut donner naissance à de l'acide chlorhydrique et il est donc important d'utiliser des écrans transparents qui résistent bien à l'acide chlorhydrique.

Selon une caractéristique de l'invention, on utilise de préférence, des films en polyuréthane, en polyméthylpentène ou en silicone qui ont une bonne résistance à l'acide chlorhydrique. De plus, ces films utilisés sous une épaisseur de l'ordre de 25 μ sont microporeux et ont une perméabilité à la vapeur d'eau au moins égale à 100 g/m²/24H/38 °C/90 % HR et ils peuvent donc être fixés de façon étanche à la plaquette-support par thermoscellage, par thermosoudage, par collage ou par enduction, le passage de la vapeur d'eau se faisant à travers le film.

## Revendications

1. Indicateur de stérilisation en présence de vapeur d'eau sous pression du type comportant une plaquette-support (1), dont au moins l'une des faces porte une ou plusieurs taches (2) d'un réactif qui change de couleur en fonction de la température, de la pression, de la concentration en vapeur d'eau et de la durée de séjour dans un stérilisateur et comportant, en outre, un écran (3) constitué par un film transparent qui est imperméable aux liquides engendrés par l'opération de stérilisation et qui recouvre entièrement lesdites taches de réactif, caractérisé en ce que ledit écran transparent (3) est constitué par un film complexe composé d'une feuille de polypropylène ou de polyuréthane à l'intérieur et d'une feuille de polyester ou de polyamide à l'extérieur, lequel film est fixé par thermofusion, de façon discontinue et non étanche, à ladite plaquette-support, de sorte que la vapeur d'eau peut passer entre ledit écran et ladite plaquette-support.

2. Indicateur de stérilisation selon la revendication 1, dans lequel ladite plaquette-support est une bande rectangulaire (1), caractérisé en ce que ledit écran a également la forme d'une bande rectangulaire (3) qui est superposée à la face de ladite plaquette portant lesdites taches de réactif et qui est fixée à ladite face par deux de ses bords opposés.

3. Indicateur de stérilisation selon la revendication 2, caractérisé en ce que ladite bande transparente (3) a les mêmes dimensions que ladite plaquette-support et elle est fixée aux petits côtés de la plaquette-support par ses deux petits côtés (3a, 3b).

4. Indicateur de stérilisation selon l'une quelconque des revendications 1 à 3, caractérisé en ce que ladite plaquette-support et/ou ledit écran transparent sont gaufrés ou striés au moins dans la plage où se trouvent lesdites taches de réactif.

5. Indicateur de stérilisation selon l'une quelconque des revendications 1 à 4, caractérisé en ce que ladite plaquette-support est constituée par du carton ondulé très mince.

6. Indicateur de stérilisation selon l'une quelconque des revendications 1 à 5, caractérisé en ce que ledit écran transparent est constitué par un film en matière plastique thermosoudé qui comporte des alvéoles qui épousent le contour desdites taches, et des canaux qui font communiquer les alvéoles avec l'atmosphère du stérilisateur.

7. Indicateur de stérilisation selon l'une quelconque des revendications 1 à 6, caractérisé en ce que ledit écran transparent est fixé à ladite plaquette-support de façon discontinue telle que la perméabilité à la vapeur d'eau soit au moins égale à 100 g de vapeur d'eau par m² et par 24 heures mesurée à une température de 38 °C, dans une atmosphère ayant une humidité relative de 90 %.

## Claims

1. Indicator of sterilization conducted in the presence of pressurized steam of the type comprising a support-plate (1) of which at least one of the faces contains one or more spots (2) of a reagent changing color in relation to the temperature, pressure, steam concentration and dwelling time in a sterilizer, and further comprising, a screen (3) constituted by a transparent film which is impervious to the liquids resulting from the sterilization operation and which entirely covers the said spots of reagent, characterized in that said transparent screen (3) is constituted by a complex film composed of a sheet of polypropylene or polyurethane on the inside and of a sheet of polyester or of polyamide on the outside, which film is bonded by melting, in discontinuous and leaky manner, to said support-plate, so that the steam can infiltrate between said screen and said support-plate.

2. Sterilization indicator according to claim 1, in which said support-plate is a rectangular strip (1), characterized in that said screen also has the shape of a rectangular strip (3) superposed on the face of said plate containing the said spots of reagent and secured to said face by two of its opposite edges.

3. Sterilization indicator according to claim 2, characterized in that said transparent strip (3) has the same dimensions as said support-plate and is fixed to the small sides of the support-plate by its two small sides (3a, 3b).

4. Sterilization indicator according to any one of claims 1 to 3, characterized in that said support-plate and/or said transparent screen are grooved or ribbed at least on the surface containing said sports of reagent.

5. Sterilization indicator according to any one of claims 1 to 4, characterized in that said support-plate is constituted by very thin corrugated cardboard.

6. Sterilization indicator according to any one of claims 1 to 5, characterized in that said transparent screen is constituted by a heat-sealed plastic film comprising alveoli which adopt the outline of said spots, and channels connecting said alveoli with the atmosphere of the sterilizer.

7. Sterilization indicator according to any one of claims 1 to 6, characterized in that said transparent screen is bonded to said support-plate in discontinuous manner so that imperviousness to steam is at least equal to 100 g of steam per square metre and per 24 hours, said imperviousness being measured at a temperature of 38 °C, in an atmosphere with 90 % relative humidity.

**Patentansprüche**

1. Sterilisationsindikator in Gegenwart von Druckwasserdampf, mit einem Plattenträger (1), von dem zumindest eine Seite einen oder mehrere Fleck(en) (2) eines Reagens trägt, welches in Abhängigkeit von der Temperatur, dem Druck, der Wasserdampfkonzentration und der Verweilzeit in einem Sterilisator die Farbe wechselt, und weiters mit einem Schild (3), welcher durch einen transparenten Film gebildet ist, der für die durch den Sterilisationsvorgang erzeugten Flüssigkeiten undurchlässig ist und die Reagensflecken vollkommen bedeckt, dadurch gekennzeichnet, daß der transparente Schild (3) durch einen zusammengesetzten, aus einer Polypropylen- oder Polyurethanfolie im Inneren und einer Polyester- oder Polyamidfolie an der Außenseite bestehenden Film gebildet ist, welcher Film mittels Wärmeschweißung diskontinuierlich und nicht dicht auf dem Plattenträger derart fixiert ist, daß der Wasserdampf zwischen dem Schild und dem Plattenträger durchtreten kann.

2. Sterilisationsindikator nach Anspruch 1, bei welchem der Plattenträger aus einem rechteckigen Band (1) besteht, dadurch gekennzeichnet, daß der Schild ebenfalls die Form eines rechteckigen Bandes (3) hat, welches auf der die Reagensflecken tragenden Seite der Platte aufgebracht und mittels zweier seiner gegenüberliegenden Ränder auf dieser Seite fixiert ist.

3. Sterilisationsindikator nach Anspruch 2, dadurch gekennzeichnet, daß das transparente Band (3) die gleichen Dimensionen wie die Trägerplatte aufweist und mit seinen beiden Schmalseiten (3a, 3b) an den Schmalseiten des Plattenträgers fixiert ist.

4. Sterilisationsindikator nach einem der Ansprüche 1 bis 3, dadurch gekennzeichnet, daß der Plattenträger und/oder der transparente Schild zumindest in dem Bereich, wo sich die Reagensflecken befinden, gerippt oder gerillt sind.

5. Sterilisationsindikator nach einem der Ansprüche 1 bis 4, dadurch gekennzeichnet, daß der Plattenträger aus sehr dünnem Wellkarton besteht.

6. Sterilisationsindikator nach einem der Ansprüche 1 bis 5, dadurch gekennzeichnet, daß der transparente Schild durch einen Film aus heißgesiegeltem Plastikmaterial gebildet ist, welcher Zellen, die an die Konturen der Flecken angepaßt sind, und Kanäle, welche die Zellen mit der Atmosphäre des Sterilisators kommunizieren lassen, aufweist.

7. Sterilisationsindikator nach einem der Ansprüche 1 bis 6, dadurch gekennzeichnet, daß der transparente Schild diskontinuierlich so auf dem Plattenträger fixiert ist, daß die Wasserdampfdurchlässigket in einer Atmosphäre mit einer relativen Feuchte von 90 % mindestens 100 g Wasserdampf pro m² und pro 24 h, gemessen bei einer Temperatur von 38 °C, beträgt.

Fig-1

Fig-2

Fig-3

Fig-4

Fig-5

Fig-6

Fig-7

1